# FASCICULE DE BREVET EUROPEEN

(11) **EP 3 449 252 B1**
(45) Date de publication et mention de la délivrance du brevet: **06.05.2020**
(21) Numéro de dépôt: 17713735.3
(22) Date de dépôt: 08.03.2017
(51) Int. Cl.: G01N 33/50

(54) **PROCÉDÉ D'ÉVALUATION DE LA CAPACITÉ D'UNE COMPOSITION À PRÉVENIR LA FATIGUE ET LES DOMMAGES MUSCULAIRES**
VERFAHREN ZUR BEURTEILUNG DER FÄHIGKEIT EINER ZUSAMMENSETZUNG ZUR PRÄVENTION VON MUSKELSCHÄDIGUNG UND -ERMÜDUNG
METHOD FOR EVALUATING THE ABILITY OF A COMPOSITION TO PREVENT MUSCLE DAMAGE AND FATIGUE

(30) Priorité: 27.04.2016 FR 1653737
(43) Date de publication de la demande: 06.03.2019
(60) Demande divisionnaire: 19185565.9
(73) Titulaire: Société d'Exploitation de Produits pour les Industries Chimiques SEPPIC, 75321 Paris cedex 07 (FR)
(72) Inventeur: CATTUZZATO, Laetitia, 81100 Castres (FR); KERN, Catherine, 81100 Castres (FR); DE POOTER, Ambre, 81100 Castres (FR)
(74) Mandataire: Laigneau, Amandine
(86) Numéro de dépôt international: PCT/FR2017/050508
(87) Numéro de publication internationale: WO 2017/187033

(56) Documents cités:
- DE-U1- 20 012 510
- ZA-B- 200 300 639
- ROGER D. HURST ET AL: "Blueberry fruit polyphenolics suppress oxidative stress-induced skeletal muscle cell damage in vitro", MOLECULAR NUTRITION & FOOD RESEARCH, vol. 54, no. 3, 1 mars 2010 (2010-03-01), pages 353-363, XP055328120, DE ISSN: 1613-4125, DOI: 10.1002/mnfr.200900094 cité dans la demande
- JANE OWENS ET AL: "Characterization of primary human skeletal muscle cells from multiple commercial sources", IN VITRO CELLULAR & DEVELOPMENTAL BIOLOGY. ANIMAL., vol. 49, no. 9, 17 juillet 2013 (2013-07-17), pages 695-705, XP055328210, New York ISSN: 1071-2690, DOI: 10.1007/s11626-013-9655-8
- JOSEF FINSTERER: "Biomarkers of peripheral muscle fatigue during exercise", BMC MUSCULOSKELETAL DISORDERS, BIOMED CENTRAL, LONDON, GB, vol. 13, no. 1, 8 novembre 2012 (2012-11-08), page 218, XP021135740, ISSN: 1471-2474, DOI: 10.1186/1471-2474-13-218 cité dans la demande
- NATASA NIKOLIC ET AL: "Electrical Pulse Stimulation of Cultured Human Skeletal Muscle Cells as an In Vitro Model of Exercise", PLOS ONE, vol. 7, no. 3, 22 mars 2012 (2012-03-22), page e33203, XP055328129, DOI: 10.1371/journal.pone.0033203
- Anonymous: "VITILICAPS", , 2013, XP055408372, Extrait de l'Internet: URL:http://www.lyconcepts.com/vitilicaps [extrait le 2017-09-20]
- ANONYMOUS: "Fall/Winter Issue 2013: SUPPORT HEART HEALTH WITH OMEGA OILS! PAGE 38 Join the BIOVEA Community on FACEBOOK! Strengthen and Improve Your Skin SIMPLE WAYS TO PRODUCTS DESIGNED SPECIFICALLY TO KEEP YOU SLIM AND FIT! FITNESS SECTION!", BIOVEA, no. Fall/Winter, 1 janvier 2013 (2013-01-01), pages 1-100, XP055408548, UK
- Ambre De Pooter ET AL: "In vitro protective effect of the association of wine polyphenols, vitamin E and zinc on muscle fatigue and damages induced by physical exercise", , 29 juin 2016 (2016-06-29), XP055367850, Extrait de l'Internet: URL:https://www.researchgate.net/publicati on/309401577_In_vitro_protective_effect_of _the_association_of_wine_polyphenols_vitam in_E_and_zinc_on_muscle_fatigue_and_damage s_induced_by_physical_exercise [extrait le 2017-04-26]
- Anonymous: "Journal of ISANH Volume 3: Special Issue for Porto Polyphenols 2016", Journal of ISANH, 29 juin 2016 (2016-06-29), XP055369257, Extrait de l'Internet: URL:http://journal.isanh.net/issue/view/38 [extrait le 2017-05-04]

## Description

L'invention concerne un procédé *in vitro* de tests visant à sélectionner une substance chimique ou une composition chimique destinée à prévenir et/ou à réduire la fatigue musculaire et les dommages musculaires induits par un effort physique chez l'être humain.

Toute période d'activité musculaire intense ou prolongée peut causer chez l'être humain une baisse de performance des muscles considérée comme de la faiblesse musculaire. La faiblesse musculaire peut concerner tous les muscles du corps, et plus particulièrement les muscles squelettiques comme par exemple les muscles des membres. Selon son intensité, la faiblesse musculaire se caractérise par une simple « fatigue musculaire » ou bien par une pathologie liée à une perte de force musculaire, à une difficulté à mobiliser les muscles et, dans le cas des muscles des membres moteurs, par une difficulté à se mouvoir.

Par définition, si la faiblesse musculaire est réversible dans une brève durée, pouvant aller de quelques minutes à quelques heures, elle est décrite comme une fatigue musculaire, soit, selon la définition de Scherrer et Monod, concernant l'activité physique «une baisse transitoire de la capacité de travail du muscle, consécutive à l'activité musculaire, survenant pour un niveau constant d'incitation des centres moteurs réversibles par le repos [Scherrer et al., « le travail musculaire local et la fatigue chez l'homme » ; J. Physiol. (Paris) 1960 ; 52 :419-501].

Par contre, lorsque la faiblesse musculaire est lentement ou difficilement réversible, et associée à des changements structuraux dans le muscle, elle est considérée comme un dommage musculaire induit par l'exercice (EIMD pour « Exercice-Induced Muscle Damage » en langue anglaise). Les dommages musculaires provoquent une faiblesse immédiate du muscle qui peut se prolonger pendant plusieurs jours avant de disparaître. De plus, du fait de ces dommages musculaires, les muscles concernés peuvent gonfler, devenir endoloris et/ou rigides pendant une durée de quelques jours après la réalisation d'un exercice physique inhabituel ou excessif pour le sujet ; ce phénomène est dénommé « douleur musculaire d'apparition retardée », (DOMS pour « Delayed Onset Muscle Soreness » en langue anglaise) [Allen DG et al., « Mechanisms of stretch-induced muscle damage in normal and dystrophic muscle: role of ionic changes » ; J. Physiol. 2005 Sep 15; 567(Pt 3): 723-35].

De manière générale, la fatigue musculaire est considérée comme étant la résultante d'un manque en énergie et en métabolites clés qui permettent aux contractions musculaires de répondre à la demande croissante d'énergie. Après une activité physique prolongée, les muscles sont fatigués et ne peuvent plus se contracter, même si le système nerveux leur demande de le faire [Baird MF et al., « Creatine-kinase- and exercise-related muscle damage implications for muscle performance and recovery » ; J. Nutr. Metab. 2012; Epub 2012 Jan 11]. Il existe plusieurs mécanismes biologiques associés à la fatigue musculaire. Les plus importants sont l'acidose musculaire et la déplétion en ATP dues à une consommation accrue ou à un manque de substrats permettant de produire de l'ATP, comme par exemple le glucose, le glycogène ou le glucose sanguin. L'acidose musculaire provient de la production d'acide lactique, qui survient au cours de la glycolyse en absence d'oxygène. Cet acide lactique est rapidement transformé en lactate et en ion hydronium. Une proportion du lactate diffuse en dehors de la cellule musculaire et se retrouve dans le sang ; cette proportion de lactate est nommée lactate sérique. La production d'ions hydroniums induit une diminution du pH et l'apparition de l'acidose musculaire, qui interfère avec les mécanismes de contractions musculaires.

Ces mécanismes associés à la fatigue musculaire peuvent être suivis par de nombreux marqueurs biologiques comme par exemple le lactate, l'ammoniaque, les oxypurines comme par exemple l'hypoxanthine et la xanthine. Le lactate sérique est le marqueur biologique de la fatigue musculaire le plus connu, car l'augmentation de sa teneur dans le sang montre que la production d'ATP en aérobie est insuffisante et nécessite d'être supplémentée avec sa production anaérobique.

De plus, il a été observé que le lactate sérique augmente avec l'intensité de l'exercice [Finsterer J, « Biomarkers of peripheral muscle fatigue during exercise » ; BMC Musculoskelet Disord., 2012; 13:218]. Les muscles squelettiques en contractions libèrent également des myokines, qui sont des cytokines produites par le muscle. Parmi ces myokines, on peut citer les interleukines 6, 8 et 15 (IL-6, IL-8, IL-15), le facteur neurotrophique issu du cerveau (BDNF pour « Brain-Derived Neurotrophic Factor »), le facteur inhibiteur de leucémie (LIF pour « Leukaemia Inhibitory Factor»), le facteur de croissance des fibroblastes (FGF-21 pour « Fibroblast Growth Factor 21 ») et la protéine apparentée à la follistatine (FSTL-1 pour « Follistatin-like 1 »).

Parmi ces myokines, il est connu que la production d'IL-6 augmente de manière exponentielle en réponse aux contractions musculaires. Elle est corrélée à la durée de l'exercice, son intensité, l'engagement des muscles, ainsi que la capacité d'endurance du sujet.

Durant l'exercice physique, l'IL-6 semble agir comme une hormone afin de mobiliser les substrats extracellulaires ou d'augmenter l'approvisionnement en substrats, comme par exemple du glucose [Finsterer J. « Biomarkers of peripheral muscle fatigue during exercise. BMC Musculoskelet Disord » ; 2012;13:218].

Absente ou présente à de très faibles quantités chez le sujet sain au repos, la concentration d'IL-6 augmente au cours de l'exercice physique et à des niveaux très élevés [Febbraio MA et al., « Muscle-derived interleukin-6: mechanisms for activation and possible biological roles » ; FASEB J. 2002; 16: 1335-47]. Des niveaux plasmatiques élevés d'IL-6 ont été associés à une sensation de fatigue accrue pendant des exercices physiques, résultant en une réduction significative de la performance chez des coureurs entrainés [Robson-Ansley PJ et al., « Acute interleukin-6 administration impairs athletic performance in healthy, trained male runners » ;Can J Appl Physiol. 2004 Aug;29(4):411-8].

La réponse IL-6 à un exercice physique est atténuée par la consommation de carbohydrates ou des régimes riches en carbohydrates avant l'exercice. Il a donc été proposé que l'IL-6 agisse comme une hormone régulant le glucose au cours de l'exercice physique et est libérée par les muscles ou le foie pour maintenir l'homéostasie glycémique.

Des exercices physiques inhabituels ou vigoureux peuvent initier des blessures ou des dommages musculaires de différentes intensités (EIMD) tels que définis précédemment. Les blessures musculaires sont caractérisées par des anomalies structurelles comme des désordres au niveau des sarcomères et des dommages membranaires, induisant la libération de composants cellulaires, l'augmentation de la dégradation des protéines musculaires et la perméabilité cellulaire, ainsi que des processus inflammatoires, dont la libération de cytokines et l'infiltration par des cellules phagocytaires [Allen DG et al., « Skeletal muscle fatigue: cellular mechanisms » ; Physiol Rev. 2008; 88: 287-332 ; Baird MF et al. J. Nutr. Metab. 2012; Epub 2012 Jan 11.] Les dommages musculaires induisent une cascade d'événements aboutissant à la douleur immédiate ou retardée dans le temps. La douleur musculaire se réfère à la douleur immédiate perçue par le sujet pendant ou juste après avoir réalisé des exercices physiques. La douleur musculaire est associée à de la raideur musculaire, à des douleurs gênantes et/ou à de la sensibilité musculaire. Ces symptômes ne sont ressentis que pendant quelques heures et sont relativement transitoires comparés à ceux des douleurs retardées (DOMS) tels que décrits précédemment.

Les symptômes en sont les mêmes que précédemment décrits mais leur apparition est retardée de 24 heures après la fin des exercices physiques. Ils sont maintenus pendant 72 heures et se résorbent lentement dans les 5 à 7 jours qui suivent [Lewis PB et al. « Muscle soreness and delayed-onset muscle soreness" Clin. Sports Med. 2012; 31: 255-62].

Les douleurs retardées sont aussi considérées comme les formes les plus communes et récurrentes de blessures sportives [Cheung K et al., « Delayed onset muscle soreness: treatment stratégies and performance factors » Sports Med. 2003; 33: 145-64].

La libération des protéines des myofibres dans le sang peut apparaître à plusieurs étapes tout au long du continuum allant des dommages musculaires à la douleur musculaire. Les marqueurs biologiques connus des dommages musculaires sont par exemple la créatine kinase, la lactate déshydrogénase. Parmi ces marqueurs, la créatine kinase est une enzyme clé qui est libérée dans la circulation sanguine quand les dommages musculaires surviennent. La créatine kinase présentant la caractéristique d'être presque exclusivement présente dans le tissu musculaire, elle est couramment dosée dans le sérum afin d'évaluer les dommages et les blessures musculaires [Baird MF et al. « Creatine-kinase- and exercise-related muscle damage implications for muscle performance and recovery. J Nutr Metab. 2012; Epub 2012 Jan 11].

De façon à prévenir ou à traiter les effets liés à la fatigue musculaire et aux dommages musculaires, il est nécessaire de mettre au point des substances chimiques ou des compositions chimiques efficaces, de façon à les administrer au sujet susceptible de produire un effort physique ou au sujet ayant produit un effort physique tel qu'il a généré l'apparition d'une fatigue physique et d'un dommage musculaire.

L'administration de telles substances chimiques ou des compositions chimiques s'effectue plus particulièrement par voie orale ou parentérale, et encore plus particulièrement par voie orale sous la forme de comprimés, de gélules, de granulés, de capsules molles en tant que compléments alimentaires.

L'administration par voie orale de telles substances chimiques ou compositions chimiques peut également prendre la forme d'une composition alimentaire comprenant lesdites substances chimiques ou des compositions chimiques.

La mise au point de substances chimiques ou de compositions chimiques efficaces pour la prévention et le traitement des effets liés à la fatigue musculaire et aux dommages musculaires nécessite un processus de recherche long et coûteux car il implique une démarche itérative entre les phases de préparation des substances ou des compositions et les phases d'évaluation des performances biologiques desdites substances ou compositions.

Pour améliorer la productivité de ce processus, des méthodes d'évaluation des performances biologiques utilisables en moyen débit sont préférables pour cribler et sélectionner les substances chimiques et les compositions chimiques performantes, ce qui implique de ne pas mettre en œuvre des méthodes d'évaluation impliquant des modèles d'études cliniques, ou impliquant des modèles de biopsies obtenues au cours d'études cliniques.

Par ailleurs, les méthodes d'évaluations biologiques mettant en œuvre des modèles sur cellules animales, ou impliquant des animaux, sont moins pertinentes car le métabolisme des animaux est différent de celui de l'homme.

Il existe donc un besoin de mettre au point une méthode de sélection d'une substance chimique ou d'une composition chimique permettant de prévenir ou de réduire la fatigue musculaire et les dommages musculaires induits par un effort physique chez l'être humain, qui soit une méthode *in vitro,* soit susceptible d'être utilisable en moyen débit dans une démarche de criblage, qui n'implique pas de cellules animales, qui soit reproductible, sensible, discriminante et efficace, notamment en permettant d'évaluer de façon concomitante les effets des substances ou compositions testées sur la fatigue musculaire et sur les dommages musculaires, et enfin qui soit adaptée au marché de la nutrition humaine.

Par « criblage », on désigne au sens de la présente demande la mise en œuvre de techniques visant à étudier, identifier, trier des molécules biologiquement actives. Dans cette démarche, seules les molécules présentant une activité pertinente selon un ou plusieurs tests, sont sélectionnées.

Les études cliniques chez l'homme sont réalisées notamment chez des sportifs en phase d'entraînement ou chez des personnes à qui est imposé un cycle d'exercice physique. Ces méthodes sont pertinentes pour évaluer l'efficacité d'un produit mais ne sont pas accessibles pour cribler et sélectionner plusieurs ingrédients. Beijer *et al.* ont par exemple étudié l'effet de vibrations additionnelles pendant une période d'entraînement de six semaines sur la réponse angiogénique chez vingt-six sujets masculins. Les sérums ont été collectés afin de doser MMP-2, MMP-9, VEGF et l'endostatine, marqueurs biologiques de l'angiogénèse, ainsi que la capacité proliférative des cellules endothéliales [Beijer Å et al., "Whole-body vibrations do not elevate the angiogenic stimulus when applied during résistance exercise" ; PLoS One. 2013 Nov. 15; 8(11): e80143.].

D'autres références bibliographiques font état d'études cliniques au cours desquelles des biopsies de muscles ont été réalisées.

Par exemple, une étude clinique a été réalisée chez 13 sujets sédentaires chez lesquels ont été évaluées la consommation d'oxygène et la sensibilité à l'insuline avant et après une période d'entraînement physique imposée à 6 semaines. Sur 8 d'entre eux, des biopsies de muscles ont été réalisées avant et après un exercice de pédalage en régime stable de 60 minutes à une consommation d'oxygène d'environ 65%.

Cette étude a permis d'analyser la capacité oxydative et le contenu en triglycérides intramusculaires et en périlipines 2 et 5 dans les biopsies [Shepherd SO et al., « Résistance training increases skeletal muscle oxidative capacity and net intramuscular triglycéride breakdown in type I and II fibres of sedentary maies » ; Exp. Physiol. 2014 Jun. ; 99(6): 894-908].

Une autre étude clinique a étudié la physiologie de la protéine irisine chez des sujets sains à différents âges et à différents niveaux d'exercice physique [Huh JY et al., « Exercise-induced irisin secretion is independent of age or fitness level and increased irisin may directly modulate muscle metabolism through AMPK activation » ; J. Clin. Endocrinol. Metab. 2014 Nov. ; 99(11): E2154-61]. L'irisine a été proposée comme myokine intervenant dans l'effet de l'exercice sur le tissu adipeux brun. Pour mieux comprendre son rôle, les auteurs ont dosé l'irisine chez des sujets âgés et jeunes, physiquement actifs ou sédentaires, après un exercice physique d'un sujet sur un tapis de course. Ils ont également réalisé des biopsies chez des sujets jeunes, modérément entraînés, avant et après un entraînement de course de huit semaines. Ces échantillons ont permis une analyse des modulations d'expression génique induite par l'exercice physique.

D'autres références bibliographiques font également état d'étude sur l'effet de l'exercice physique chez l'animal. On peut notamment retenir une étude concernant l'effet bénéfique de l'exercice physique sur la sarcopénie [Cisterna B et al. « Adapted physical exercise enhances activation and differentiation potential of satellite cells in the skeletal muscle of old mice" ; J. Anat. 2016 Jan 6. doi: 10.1111/joa.12429]. Dans ce cadre, la quantité et l'activation des cellules satellites (cellules contributrices dans la régénération des muscles), ainsi que leur potentiel de prolifération et de différenciation, ont été évalués *in situ* chez des souris âgées de 28 mois soumises à un exercice physique sur tapis de course. Cette étude a permis de montrer que l'exercice physique est une approche puissante et non-pharmacologique pour lutter contre la sarcopénie et la détérioration des cellules satellites liée à l'âge.

De nombreuses références bibliographiques relatives aux études *in vitro* portent sur des méthodes mises en œuvre sur les cellules musculaires murines, et notamment les cellules des lignées murines C2C12 et L6, qui sont des lignées de myoblastes de souris et de rats respectivement, capables de se différencier rapidement pour former des myotubes capables de se contracter et de produire des protéines musculaires.

A ce titre, on peut citer :
- Une étude relative à l'effet de la protéine irisine sur le métabolisme, l'expression génique et le contenu mitochondriale des myocytes C2C12 [Vaughan RA et al., « Characterization of the metabolic effects of irisin on skeletal muscle in vitro » ; Diabetes Obes. Metab. 2014 Aug;16(8): 711-8] ;
- Une étude relative aux mécanismes d'apoptose, essentielle au développement et à l'homéostasie du muscle squelettique sur myoblastes et myotubes C2C12 exposés aux rayonnements UV-B, ou à l'hypo- et l'hyperthermie, ou à un faible pH [Battistelli M et al., « Skeletal Muscle Cell Behavior After Physical Agent Treatments » ; Curr. Pharm. Des. 2015; 21(25): 3665-72] ;
- Une étude relative à la réponse fonctionnelle de myotubes, mettant en œuvre une méthode comprenant des étapes de prélèvement et d'isolation de myotubes de rats, suivi d'une étape de traitement avec la créatine ou d'une étape de stimulation électrique pour simuler un exercice physique chronique [McAleer CW et al., « Mechanistic investigation of adult myotube response to exercise and drug treatment in vitro using a multiplexed functional assay system » ; J. Appl. Physiol. (1985). 2014 Dec 1; 117(11):1398-405] ;
- Une étude mettant en œuvre une méthode d'évaluation impliquant des cellules de la lignée musculaire L6 de rats, comprenant une étape de stimulation de ladite lignée musculaire, afin de mimer le stress associé à un exercice physique, par l'ajout de l'ionophore de calcium A23187 ; cette étude a permis de montrer un effet protecteur des polyphénols compris dans des extraits de myrtilles sur la génération d'espèces réactives de l'oxygène, ainsi que sur la libération des enzymes lactate déshydrogénase et créatine kinase [Hurst RD et al., « Blueberry fruit polyphenolics suppress oxidative stress-induced skeletal muscle cell damage in vitro » ; Mol. Nutr. Food Res. 2010 Mar; 54(3): 353-63] ;
- D'autres références bibliographiques relatives aux études *in vitro* portent sur des méthodes mettant en jeu des cellules musculaires squelettiques humaines. A ce titre, on peut citer une étude mettant en œuvre une méthode d'évaluation impliquant des cellules musculaires squelettiques humaines prélevées chez les sujets afin d'étudier la consommation de glucose et d'acides gras par l'ajout d'irisine dans le milieu de culture, ainsi que l'induction de l'expression de gènes liés au métabolisme du glucose, du glycogène et des lipides sur ces mêmes cellules stimulées par l'irisine [Huh JY et al., « Exercise-induced irisin secretion is independent of age or fitness level and increased irisin may directly modulate muscle metabolism through AMPK activation » ; J. Clin. Endocrinol. Metab. 2014 Nov; 99(11): E2154-61] ;
   Il apparaît que l'état de la technique ne divulgue pas de méthode *in vitro* de sélection d'une substance chimique ou d'une composition chimique permettant de prévenir de façon concomitante la fatigue musculaire et les dommages musculaires induits par un effort physique chez l'être humain, qui soit susceptible d'être utilisable en moyen débit dans une démarche de criblage, n'implique pas de cellules animales, soit reproductible, sensible, discriminante et efficace, et soit adapté au marché de la nutrition humaine.

Plus particulièrement, l'état de la technique ne divulgue ni n'enseigne de telles méthodes de sélection impliquant des cellules musculaires squelettiques primaires humaines et comprenant une étape de stimulation par l'ajout d'un agent ionophore.

Par conséquent, l'absence de procédés de tests *in vitro* adaptés et efficaces, susceptibles d'être utilisables en moyen débit, entrave le développement de compléments alimentaires ou d'aliments efficaces permettant de prévenir de façon concomitante la fatigue musculaire et les dommages musculaires induits par un effort physique chez l'être humain.

Selon un premier aspect, l'invention a pour objet un procédé pour évaluer la capacité d'une substance chimique (S) ou d'une composition chimique (C) à prévenir la fatigue musculaire et les dommages musculaires induits par un effort physique chez l'être humain, ledit procédé étant caractérisé en ce qu'il comprend :
- Une étape a) de mise en culture de cellules musculaires squelettiques primaires humaines ;
- Une étape b) de différenciation des cellules obtenues à l'étape a) pour obtenir des myotubes ;
- Une étape c) de mise en contact d'une substance chimique (S) ou d'une composition chimique (C) avec le milieu cellulaire obtenu à l'étape b) ;
- Une étape d) de mise en contact d'au moins un agent ionophore de calcium (Al) avec le milieu cellulaire obtenu à l'étape c) ;
- Une étape e) de mesure du niveau d'expression d'une combinaison de marqueurs biologiques comprenant ou consistant en :
- Au moins un marqueur biologique (M₁) de l'homéostasie glycémique choisi parmi les éléments du groupe constitué par les myokines ou cytokines produites par les muscles,
- Au moins un marqueur biologique (M₂) des lésions musculaires choisi parmi la créatine kinase et la lactate déshydrogénase,
- Au moins un marqueur biologique (M₃) du métabolisme de l'ATP choisi parmi les éléments du groupe constitué par le lactate, l'ammoniaque, les oxypurines, dans le milieu de culture obtenu à l'étape d) ; et
- Une étape f) de comparaison des niveaux d'expression de chacun desdits trois marqueurs biologiques (M₁), (M₂) et (M₃) mesurés à l'étape e) avec le niveau d'expression de référence pour chacun de ces trois marqueurs biologiques.

Par «capacité à prévenir la fatigue musculaire et les dommages musculaires», on entend la capacité dans le cadre de la présente invention, la capacité à diminuer la durée pendant laquelle s'observe une baisse de la performance des muscles liée à une faiblesse musculaire après la réalisation d'un effort physique et à diminuer l'intensité des changements structuraux dans le muscle pouvant intervenir après la réalisation d'un effort physique.

Par « effort physique », on entend dans le cadre de la présente invention, le fait de mobiliser et de mettre en œuvre ses forces physiques en vue de vaincre une résistance, et/ou d'atteindre une performance, comme réaliser des mouvements du corps et de ses membres à l'aide de la contraction de ses muscles.

Par « cellule musculaire squelettique primaire », on désigne une cellule qui est directement extraite d'un muscle squelettique.

Par « myotube », on désigne une cellule plurinucléée, formée par la fusion de plusieurs myoblastes lors de la myogenèse. Les myotubes se différencient ensuite en fibres musculaires. Un myoblaste est une cellule souche responsable de la formation des muscles squelettiques.

Par « agent ionophore », on entend au sens de la présente invention, un « transporteur d'ions », capable de catalyser le transport d'ions à travers des membranes hydrophobiques telles que les bicouches lipidiques des cellules vivantes ou des vésicules. En particulier, un ionophore de calcium est capable de faire entrer des ions calcium dans les cellules musculaires, mimant ainsi un exercice physique. Parmi les agents ionophores de calcium (Al) mis en œuvre dans l'étape d) du procédé objet de la présente invention, on peut citer plus particulièrement, la beauvéricine, l'ionomycine, la calcimycine (ou A23187) et tout particulièrement la calcimycine (ou A23187).

Par « marqueur biologique », on entend au sens de la présente invention, une caractéristique qui est objectivement mesurée et évaluée comme indicateur de processus biologiques normaux, de processus pathogéniques, ou de réponse pharmacologiques à une intervention extérieure. Un marqueur biologique peut être par exemple une substance dont la détection indique un état pathologique particulier ou au contraire une substance dont la détection indique un état physiologique particulier.

Par « marqueur biologique (M₁) de l'homéostasie glycémique », on désigne un marqueur biologique tel que défini précédemment, dont la variation d'expression est corrélée avec l'homéostasie glycémique. Parmi les marqueurs biologiques (M₁) de l'homéostasie glycémique dont on mesure le niveau d'expression dans l'étape e) du procédé objet de la présente invention, on peut citer plus particulièrement l'interleukine-6 (IL-6), l'interleukine-8 (IL-8), l'interleukine-15 (IL-15), le facteur neurotrophique issu du cerveau (BDNF pour « Brain-Derived Neurotrophic Factor »), le facteur inhibiteur de leucémie (LIF pour « Leukaemia Inhibitory Factor ») et tout particulièrement l'interleukine-6,

Par « homéostasie glycémique », on la capacité d'un organisme à maintenir son équilibre glycémique, quelque soient les contraintes externes, en particulier au cours d'un effort physique. Le glucose est en mouvement entre ses sites d'absorption (muqueuse intestinale) ou de production endogène (foie) et ceux de son métabolisme à visée essentiellement énergétique, et notamment les muscles squelettiques lors d'un effort physique.

Au sens de la présente demande, le marqueur biologique IL-6 comprend le gène IL-6 humain (référence NCBI : Gene ID : GenBank: JQ250825.1), ainsi que les produits de ce gène. Dans un mode de réalisation particulier, le marqueur biologique IL-6 consiste en l'un des produits du gène IL-6 humain. Les produits du gène IL-6 humain comprennent le transcrit du gène IL-6 humain et la protéine humaine IL-6. Au sens de la présente demande, le « transcrit du gène IL-6 humain » est le polynucléotide dont la séquence a pour référence NCBI : GenBank: M54894.1. Par « protéine humaine IL-6 », on entend au sens de la présente demande la protéine dont la séquence peptidique est la séquence de référence NCBI GenBank: AAD13886.1.

Par « marqueur biologique (M₂) des lésions musculaires », on désigne un marqueur biologique tel que défini précédemment, dont la variation d'expression est corrélée avec les lésions musculaires. Parmi les marqueurs biologiques (M₂) des lésions musculaires, dont on mesure le niveau d'expression dans l'étape e) du procédé objet de la présente invention, on peut citer plus particulièrement la créatine kinase et la lactate déshydrogénase.

Par « marqueur biologique (M₃) du métabolisme de l'ATP », on désigne un marqueur biologique tel que défini précédemment, dont la variation d'expression est corrélée avec le métabolisme de l'ATP. Parmi les marqueurs biologiques (M₃) du métabolisme de l'ATP, dont on mesure le niveau d'expression dans l'étape e) du procédé objet de la présente invention, on peut citer plus particulièrement le lactate, l'ammoniaque et les oxypurines.

Selon un aspect particulier du procédé tel que défini précédemment, dans l'étape e), le marqueur biologique (M₁) est l'Interleukine-6, le marqueur biologique (M₂) est la créatine kinase et le marqueur biologique (M₃) est le lactate.

Pour chacun des marqueurs biologiques (M₁), (M₂) et (M₃), l'expression « la mesure du niveau d'expression » se réfère de manière générale soit à une mesure de la quantité de transcrits d'un gène, soit à une mesure de la quantité de molécules biologiques, et plus particulièrement des protéines et des métabolites, produites par le corps humain, soit à un niveau d'activité enzymatique.

Lorsque le niveau d'expression du marqueur biologique est mesuré au niveau nucléotidique, à savoir par la mesure de la quantité de produits du gène sous sa forme nucléotidique, toute méthode habituellement mise en œuvre par l'homme du métier pour mesurer des quantités nucléotidiques peut être mise en œuvre, et on peut ainsi citer la qRT-PCR (quantitative reverse-transcription polymerase chain reaction), les puces à ADN, l'hybridation in situ.

Lorsque le niveau d'expression du marqueur biologique est mesuré au niveau protéique, fonctionnel ou métabolique, à savoir en mesurant sa quantité s'il s'agit d'une protéine ou d'un métabolite, ou en mesurant sa fonction biologique, toute méthode habituellement mise en œuvre par l'homme du métier pour mesurer des quantités de protéines ou de métabolites, ou des fonctionnalités peut être mise en œuvre et on peut ainsi citer le dosage ELISA, le Western-Blot, la spectrométrie de masse, l'immunofluorescence, des techniques chromatographiques, l'activité enzymatique.

Dans le procédé objet de la présente invention, la mesure de l'expression du marqueur biologique (M₁) de l'homéostasie glycémique est réalisée plus particulièrement au niveau protéique.

Selon un aspect plus particulier du procédé objet de la présente invention, lorsque le marqueur biologique (M₁) est l'Interleukine-6, la mesure de l'expression de l'Interleukine-6 est réalisée au niveau protéique et encore plus particulièrement avec une mesure mettant en œuvre une méthode ELISA (Enzyme-Linked ImmunoSorbent Assay) colorimétrique. Cette méthode utilise un anticorps spécifique de l'IL-6 humaine fixée au fond des puits d'une plaque de mesure. Les échantillons à doser sont ajoutés dans les puits et l'IL-6 présente dans les échantillons se fixe à l'anticorps immobilisé. Les puits sont ensuite lavés et un autre anticorps anti-IL-6 humaine biotinylé est ajouté.

Les puits sont une nouvelle fois rincés pour éliminer l'anticorps non fixé, puis de la streptavidine conjuguée à l'enzyme HRP (horseradish peroxidase) est ajoutée aux puits. Les puits sont à nouveau rincés et le substrat tetramethylbenzidine de l'HRP est ajouté aux puits. L'intensité de la couleur se développe proportionnellement à la quantité d'IL-6 liée. La solution « stop » change la couleur du bleu au jaune, et l'intensité de couleur est mesurée à 450 nm par spectrophotométrie. La concentration en IL-6 est calculée en se référant à une gamme étalon.

Dans le procédé objet de la présente invention, la mesure de l'expression du marqueur biologique (M₂) des lésions musculaires est réalisée plus particulièrement au niveau fonctionnel, et plus particulièrement au niveau activité enzymatique.

Selon un aspect plus particulier du procédé objet de la présente invention, lorsque le marqueur biologique (M₂) est la créatine kinase, la mesure de l'expression de la créatine kinase est réalisée au niveau fonctionnel, et encore plus particulièrement avec une mesure de son activité enzymatique. L'activité enzymatique de la créatine kinase est déterminée par méthode ELISA colorimétrique. L'essai est basé sur des réactions enzymatiques couplées au cours desquelles la créatine phosphate et l'ADP sont convertis en créatine et ATP par la créatine kinase présente dans l'échantillon à doser.

L'ATP ainsi produit est alors utilisé pour phosphoryler le glucose en présence d'hexokinase afin de générer du glucose-6-phosphate, ce dernier étant par la suite oxydé par le NADP en présence de glucose-6 phosphate déshydrogénase. La quantité de NADPH produit à différents temps de mesure, détecté à 340 nm, est proportionnelle à l'activité créatine kinase de l'échantillon. L'activité créatine kinase est ensuite calculée en se référant à un échantillon calibré.

Dans le procédé objet de la présente invention, la mesure de l'expression du marqueur biologique (M₃) du métabolisme de l'ATP est réalisée plus particulièrement au niveau métabolique.

Selon un aspect plus particulier du procédé objet de la présente invention, lorsque le marqueur biologique (M₃) est le lactate, la mesure de l'expression du lactate est réalisée au niveau métabolique, et encore plus particulièrement avec une mesure mettant en œuvre un dosage enzymatique colorimétrique.

Brièvement, le lactate présent dans les surnageants de culture réagit spécifiquement avec un mélange enzymatique pour générer un produit, qui interagit avec une sonde spécifique pour produire de la couleur.

La densité optique est mesurée par spectrophotométrie à 570nm et la concentration en lactate est calculée grâce à une gamme étalon.

Selon un aspect encore plus particulier du procédé objet de la présente invention, lorsque le marqueur biologique (M₁) est l'Interleukine-6, la mesure de l'expression de l'Interleukine-6 est réalisée au niveau protéique, et encore plus particulièrement avec une mesure mettant en œuvre une méthode ELISA colorimétrique, et lorsque le marqueur biologique (M₂) est la créatine kinase, la mesure de l'expression de la créatine kinase est réalisée au niveau fonctionnel, et encore plus particulièrement avec une mesure mettant en œuvre une mesure de son activité enzymatique par méthode ELISA colorimétrique, et lorsque le marqueur biologique (M₃) est le lactate, la mesure de l'expression du lactate est réalisée au niveau métabolique, et encore plus particulièrement avec une mesure mettant en œuvre un dosage enzymatique colorimétrique.

Par un « niveau d'expression de référence » d'un marqueur biologique, on désigne tout niveau d'expression dudit marqueur biologique utilisé à titre de référence.

Par exemple, un niveau d'expression de référence peut être obtenu en mesurant le niveau d'expression dudit marqueur biologique avec une substance connue de l'art antérieur pour son effet dans la prévention de la fatigue musculaire et des dommages musculaires chez l'être humain, ou en mesurant le niveau d'expression dudit marqueur biologique lorsque le procédé objet de la présente invention est mis en œuvre en l'absence de l'étape c) et de l'étape d) dudit procédé ; le niveau d'expression du marqueur biologique d'intérêt est alors celui correspondant au milieu de culture des cellules différenciées non traitées et non stressées, ou en mesurant le niveau d'expression dudit marqueur biologique lorsque le procédé objet de la présente invention est mis en œuvre en l'absence de l'étape c) dudit procédé ; le niveau d'expression du marqueur biologique d'intérêt est alors celui correspondant au milieu de culture des cellules différenciées non traitées et stressées.

Dans le cadre de ma présente invention, un niveau d'expression de référence du marqueur biologique (M₁) de l'homéostasie glycémique peut être obtenu en mesurant le niveau d'expression dudit marqueur biologique (M₁),
- Dans le milieu de culture cellulaire obtenu à l'issue de l'étape b) du procédé objet de l'invention, sans mettre en œuvre ni l'étape c) et ni l'étape d) du procédé objet de la présente invention ; cette mesure du niveau d'expression du marqueur (M₁) sur les cellules différenciées non traitées et non stressées est notée N¹, et/ou
- Dans le milieu de culture cellulaire obtenu à l'issue de la mise en œuvre des étapes a), b) et d) du procédé objet de la présente invention, mais sans mettre en œuvre l'étape c) du procédé objet de la présente invention ; cette mesure du niveau d'expression du marqueur (M₁) sur les cellules différenciées non traitées mais stressées est notée N¹₀ ; et/ou
- Dans le milieu de culture cellulaire obtenu à l'issue de la mise en œuvre de l'étape d) du procédé objet de la présente invention, lorsque la substance (S) mise en œuvre dans l'étape c) du procédé objet de la présente invention est une substance choisie parmi les éléments du groupe constitué par la vitamine E, le glucose, la cyclosporine A, l'actinomycine D, et plus particulièrement lorsque la substance (S) mise en œuvre dans l'étape c) du procédé objet de la présente invention est la vitamine E. Cette mesure du niveau d'expression du marqueur biologique (M₁) pour une substance (S) de référence est notée N¹_{Ref}.

Dans le cadre de la présente invention, un niveau d'expression de référence du marqueur biologique (M₂) des lésions musculaires peut être obtenu en mesurant le niveau d'expression dudit marqueur biologique (M₂) :
- Dans le milieu de culture cellulaire obtenu à l'issue de l'étape b) du procédé objet de l'invention, sans mettre en œuvre ni l'étape c) et ni l'étape d) du procédé objet de la présente invention ; cette mesure du niveau d'expression sur les cellules différenciées non traitées et non stressées est notée N², et/ou
- Dans le milieu de culture cellulaire obtenu à l'issue de la mise en œuvre des étapes a), b) et d) du procédé objet de la présente invention, mais sans mettre en œuvre l'étape c) du procédé objet de la présente invention ; cette mesure du niveau d'expression sur les cellules différenciées non traitées mais stressées est notée N²₀, et/ou
- Dans le milieu de culture cellulaire obtenu à l'issue de la mise en œuvre de l'étape d) du procédé objet de la présente invention, lorsque la substance (S) mise en œuvre dans l'étape c) du procédé objet de la présente invention est une substance choisie parmi les éléments du groupe constitué par la vitamine E, l'IL-1RA (interleukin-1 receptor antagonist), la dihydromyricétine, l'andrographolide, la nifédipine et plus particulièrement lorsque la substance (S) mise en œuvre dans l'étape c) du procédé objet de la présente invention est la vitamine E. Cette mesure du niveau d'expression du marqueur biologique (M₂) pour une substance (S) de référence est notée N²_{Ref}.

Dans la cadre de la présente invention, un niveau d'expression de référence du marqueur biologique (M₃) du métabolisme de l'ATP peut être obtenu en mesurant le niveau d'expression dudit marqueur biologique (M₃) :
- Dans le milieu de culture cellulaire obtenu à l'issue de l'étape b) du procédé objet de l'invention, sans mettre en œuvre ni l'étape c) et ni l'étape d) du procédé objet de la présente invention ; cette mesure du niveau d'expression du marqueur biologique (M₃) sur les cellules différenciées non traitées et non stressées est notée N³, et/ou
- Dans le milieu de culture cellulaire obtenu à l'issue de la mise en œuvre des étapes a), b) et d) du procédé objet de la présente invention, mais sans mettre en œuvre l'étape c) du procédé objet de la présente invention ; cette mesure du niveau d'expression du marqueur biologique (M₃) sur les cellules différenciées non traitées mais stressées est notée N³₀, et/ou
- Dans le milieu de culture cellulaire obtenu à l'issue de la mise en œuvre de l'étape d) du procédé objet de la présente invention, lorsque la substance (S) mise en œuvre dans l'étape c) du procédé objet de la présente invention est une substance choisie parmi les éléments du groupe constitué par la vitamine E, le glutathion, le magnésium, le coenzyme Q10, et plus particulièrement lorsque la substance (S) mise en œuvre dans l'étape c) du procédé objet de la présente invention est la vitamine E. Cette mesure du niveau d'expression du marqueur biologique (M₃) pour une substance (S) de référence est notée N³_{Ref}.

Grâce au procédé tel que défini précédemment, il est possible de sélectionner ladite substance (S) ou de ladite composition (C) comme ingrédient destiné à prévenir la fatigue musculaire et les dommages musculaires induits par un effort physique chez l'être humain, et apte à être utilisée en tant que telle, ou pour préparer des compositions de compléments nutritionnels la comprenant, ou pour préparer des aliments la comprenant, pour prévenir la fatigue musculaire et les dommages musculaires induits par un effort physique chez l'être humain.

Grâce au procédé tel que défini précédemment, une substance (S) ou une composition (C) sera sélectionnée pour être utilisée en tant que telle, ou pour préparer des compositions de compléments nutritionnels la comprenant, ou pour préparer des aliments la comprenant, pour prévenir la fatigue musculaire et les dommages musculaires induits par un effort physique chez l'être humain si :
- Le niveau d'expression mesuré pour ladite substance (S) ou pour ladite composition (C), noté N¹ᵢ, est supérieur au niveau d'expression de référence du marqueur biologique (M₁) de l'homéostasie glycémique, et si
- Le niveau d'expression mesuré pour ladite substance (S) ou pour ladite composition (C) noté N²ᵢ, est supérieur au niveau d'expression de référence du marqueur biologique (M₂) des lésions musculaires et
- Le niveau d'expression mesuré pour ladite substance (S) ou pour ladite composition (C), noté N³ᵢ, est supérieur au niveau d'expression de référence du marqueur biologique (M₃) du métabolisme de l'ATP.

Une substance (S) ou une composition (C) sera plus particulièrement sélectionnée pour être utilisée en tant que telle, ou pour préparer des compositions de compléments nutritionnels la comprenant, ou pour préparer des aliments la comprenant, pour prévenir la fatigue musculaire et les dommages musculaires induits par un effort physique chez l'être humain si :
- Le rapport R₁ = [(N¹₀ - N¹i) x 100] / [(N¹₀ - N¹)] est supérieur à n₁ ; si
- Le rapport R₂ = [(N²₀ - N²i) x 100] / [(N²₀ - N²] est supérieur à n₂, et si
- Le rapport R₃ = [(N³₀ - N³i) x 100] / [(N³₀ - N³] est supérieur à n₃,
   avec :
   n₁ supérieur à 20, et plus particulièrement supérieur à 40, n₂ supérieur à 20, et plus particulièrement supérieur à 40 et n₃ supérieur à 20 et plus particulièrement supérieur à 40, N¹, N¹₀, N², N²₀, N³ et N²₀ étant tels que définis précédemment.
   n₁ tel que défini ci-dessus est plus particulièrement supérieur ou égal à 40, tout particulièrement supérieur ou égal à 70, et encore plus particulièrement supérieur ou égal à 100.
   n₂ tel que défini ci-dessus est plus particulièrement supérieur ou égal à 40, tout particulièrement supérieur ou égal à 70, et encore plus particulièrement supérieur ou égal à 100.
   n₃ tel que défini ci-dessus est plus particulièrement supérieur ou égal à 40, tout particulièrement supérieur ou égal à 70, et encore plus particulièrement supérieur ou égal à 100.

Grâce au procédé tel que défini précédemment, une substance (S) ou une composition (C) sera plus particulièrement sélectionnée n₁, n₂ et n₃ sont supérieurs ou égaux à 40.

Selon un autre aspect, ce document a pour objet une composition comestible (C_{A}) comprenant :
- Au moins un sel d'un cation métallique multivalent (C_{METAL}),
- Au moins un composé (V_{E}), choisi parmi la vitamine E ou l'acétate de vitamine E,
- Au moins un composé polyphénolique choisi parmi les composés de la famille des flavanols, les composés de la famille des anthocyanes, les composés de la famille des acides phénoliques, et des composés de la famille des flavonols et/ou de leurs dérivés glucosylés.
   dans laquelle le ratio molaire (C_{METAL})/(V_{E}) est supérieur ou égal à 0,50 et inférieur ou égal à 2,00, plus particulièrement supérieur ou égal à 0,50 et inférieur ou égal à 1,50. Ce document a notamment pour objet ladite composition (C_{A}) telle que définie ci-dessus, dans laquelle le cation métallique multivalent (C_{METAL}) est un cation métallique divalent, et plus particulièrement un cation métallique divalent choisi parmi les cations du calcium, du magnésium, du zinc, du manganèse, du fer, du cuivre, du cobalt, de l'argent, du baryum, du zirconium et du strontium.

Selon un aspect plus particulier, le cation métallique multivalent (C_{METAL}) est un cation métallique divalent choisi les cations du calcium, du magnésium, du zinc et du strontium.

Selon un aspect tout particulier, le cation métallique multivalent (C_{METAL}) est le cation divalent du zinc.

Le cation métallique multivalent (C_{METAL}) est mis en œuvre dans la composition (C_{A}) telle que définie précédemment sous la forme d'un sel avec un anion organique comestible, possédant au moins une fonction acide carboxylique sous forme carboxylate, choisi parmi les éléments du groupe constitué par les anions issus des acides glycolique, citrique, tartrique, salicylique, lactique, mandélique, ascorbique, pyruvique, fumarique, glycérophosphorique, rétinoïque, benzoïque, kojique, malique, gluconique, galacturonique, propionique, heptanoïque, 4-amino benzoïque, cinnamique, benzalmalonique, aspartique et glutamique.

Selon un aspect particulier, le cation métallique multivalent (C_{METAL}) est mis en œuvre dans ladite composition (C_{A}) telle que définie précédemment sous la forme du gluconate, du glycérophosphate, de l'aspartate ou du glutamate dudit cation métallique multivalent (C_{METAL}).

Selon un aspect tout particulier, le cation métallique multivalent (C_{METAL}) est mis en œuvre dans ladite composition (C_{A}) sous la forme du gluconate dudit cation métallique multivalent (C_{METAL}).

Par vitamine E, on désigne, dans la définition de ladite composition (C_{A}) telle que définie précédemment :
- L'a-tocophérol ou (*2R*)-2,5,7,8-tétraméthyl-2-[(4*R*,8*R*)-4,8,12-triméthyltridécyl]-3,4-dihydrochromèn-6-ol,
- Le β-tocophérol ou (2*R*)-2,5,8-triméthyl-2-[(4*R*,8*R*)-4,8,12-triméthyltridécyl]-3,4-dihydrochromèn-6-ol,
- Le γ-tocophérol ou (2*R*)-2,7,8-triméthyl-2-[(4*R*,8*R*)-4,8,12-triméthyltridécyl]-3,4-dihydrochromèn-6-ol,
- Le δ-tocophérol ou (2*R*)-2,8-diméthyl-2-[(4*R*,8*R*)-4,8,12-triméthyltridécyl]-3,4-dihydrochromèn-6-ol,
- L'a-tocotriènol ou (2*R*)-2,5,7,8-tétraméthyl-2-[(3*E*,7*E*)-4,8,12-triméthyltridéca-3,7,11-triènyl]-3,4-dihydrochromèn-6-ol,
- Le β-tocotriènol ou (2*R*)-2,5,8-triméthyl-2-[(3*E*,7*E*)-4,8,12-triméthyltridéca-3,7,11-triènyl]-3,4-dihydrochromèn-6-ol,
- Le γ-tocotriènol ou (2*R*)-2,7,8-triméthyl-2-[(3*E*,7*E*)-4,8,12-triméthyltridéca-3,7,11-triènyl]-3,4-dihydrochromèn-6-ol et
- Le δ-tocotriènol ou (2*R*)-2,8-diméthyl-2-[(3*E*,7*E*)-4,8,12-triméthyltridéca-3,7,11-triènyl]-3,4-dihydrochromèn-6-ol.

Par α-tocophérol, β-tocophérol, γ-tocophérol, δ-tocophérol, α-tocotriénol, β-tocotriénol, γ-tocotriénol et δ-tocothénol, on désigne soit les formes diastéréoisomériquement pures de chacun desdits composés tels que précédemment énoncés, soit les racémates desdits composés, comme par exemple le DL-α-tocophérol.

Les acétates de tocophérols, comme l'acétate d'a-tocophérol, l'acétate de β-tocophérol, l'acétate de γ-tocophérol ou l'acétate de δ-tocophérol, sont des composés synthétiques obtenus à partir des tocophérols correspondants.

Selon un aspect particulier, ce document a pour objet ladite composition (C_{A}) telle que définie précédemment dans laquelle la vitamine E est choisi parmi les éléments du groupe constitué par l'α-tocophérol et l'acétate d'α-tocophérol.

Selon un aspect tout particulier, ladite composition (C_{A}) telle que définie précédemment comprend de l'acétate d'α-tocophérol.

Par « composé de la famille des flavanols », on désigne un composé dérivé d'une des structures monomériques suivantes de formule (I) : 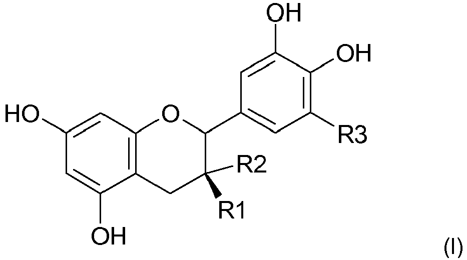 et notamment :
- La (+)-catéchine lorsque R1 = OH, R2 = H et R3 = H,
- L'(-)-épicatéchine lorsque R1 = H, R2 = OH et R3 = H,
- La (+)-gallocatéchine lorsque R1 = OH, R2 = H et R3 = OH, et
- L'(-)-épigallocatéchine lorsque R1 = H, R2 = OH et R3 = OH.

Par « composé de la famille des anthocyanes », on désigne le composé monoglucosylé ou le composé polyglucosylé, dont les aglycones (anthocyanidines) présentant une des structures de formule (II) : 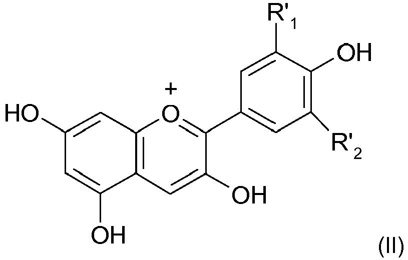 et notamment :
- La malvidine lorsque R'1 et R'2 représentent chacun un radical méthoxy,
- La péonidine lorsque R'1 représente un radical méthoxy et R'2 un atome d'hydrogène,
- La delphinidine lorsque R'1 et R'2 représentent chacun un radical hydroxy, et
- La pétunidine lorsque R'1 représente un radical méthoxy et R'2 un radical hydroxy.

Par « composé de la famille des acides phénoliques», on désigne plus particulièrement un composé choisi parmi les acides caftarique, cis-coutarique, trans-coutarique, caféique, gallique, para-coumarique ou 2-S-glutathionyl caftarique.

Par « composé de la famille des flavonols ou de leurs dérivés glucosylés », on désigne plus particulièrement les éléments du groupe constitué par le glucoside de myricétol, le glucoside de quercétol, le composé présentant une des structures de formule (III) : 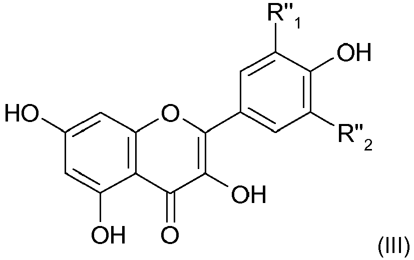 et notamment :
- Le kaempférol, lorsque R"1 et R"2 représentent chacun un atome d'hydrogène,
- La quercétine, lorsque R"1 représente un radical hydroxy et R"2 représente un atome d'hydrogène,
- La myricétine, lorsque R"1 et R"2 représentent chacun un radical hydroxy, et
- L'isorhamétine, lorsque R"1 représente un radical méthoxy et R"2 représente un atome d'hydrogène.

Selon un aspect particulier, ce document a pour objet ladite composition comestible (C_{A}) telle que définie ci-dessus et comprenant pour 100% de sa masse :
- De 5% à 50% massique, plus particulièrement de 5% à 40% massique, d'un sel d'un cation métallique multivalent (C_{M}) et d'un anion organique comestible,
- De 1% à 35% massique, plus particulièrement de 5% à 35% massique et tout particulièrement de 8% à 35% massique d'au moins un composé (V_{E}), choisi parmi la vitamine E ou l'acétate de vitamine E, d'acétate de vitamine E,
- De 0,5% à 80% massique, plus particulièrement de 1% à 70% massique d'une composition comestible (PP) de composés polyphénoliques choisis parmi les composés de la famille des flavanols, les composés de la famille des anthocyanes, les composés de la famille des acides phénoliques, et des composés de la famille des flavonols et/ou de leurs dérivés glucosylés,
- De 10% à 93,5% massique, plus particulièrement de 15% à 89% massique d'au moins un additif technologique comestible,
   et dans laquelle le rapport molaire (C_{M})/(V_{E}) est supérieur ou égal à 0,50 et inférieur ou égal à 2,00, plus particulièrement supérieur ou égal à 0,50 et inférieur ou égal à 1,50.

Selon un aspect particulier, ce document a pour objet la composition (C_{A}) telle que définie ci-dessus, dans laquelle ladite composition (PP) comprend pour 100% de sa masse :
- de 60 % à 75 % massique d'au moins un composé de la famille des flavanols,
- de 10 % à 20 % massique d'au moins un composé de la famille des anthocyanes
- de 5 % à 30 % massique d'au moins un composé de la famille des acides phénoliques.

Selon un aspect tout particulier, ce document a pour objet la composition (C_{A}) telle que définie ci-dessus dans laquelle ladite composition (PP) comprend en outre jusqu'à 5% massique d'au moins un composé de la famille des flavonols ou de leurs dérivés glucosylés.

Selon un autre aspect particulier, ce document a pour objet la composition (C_{A}) telle que définie ci-dessus dans laquelle ladite composition (PP) est un extrait de jus de raisins ou d'un résidu sec pulvérulent de vin rouge et notamment d'un résidu sec pulvérulent de vin rouge obtenu par un procédé comportant les étapes successives suivantes :
- Une étape a1) de distillation de vin rouge,
- Une étape b1) de concentration du distillât obtenu à l'étape a1), et
- Une étape c1) de séchage du concentré obtenu à l'étape b1).
Le résidu sec de vin rouge est ainsi obtenu sous forme d'une poudre contenant généralement au plus 5% massique d'eau d'humidité.

Par « additif technologique comestible, présent dans la composition (C_{A}) objet de la présente invention, on désigne toute substance chimique ou toute composition chimique dont la fonction technique est de permettre et/ou de faciliter le mélange des différents constituants de ladite composition (C_{A}), de faciliter et/ou d'optimiser les propriétés physiques de ladite composition (C_{A}), comme par exemple de faciliter et/ou d'optimiser son écoulement, sa stabilité, et son incorporation dans une formulation pharmaceutique et/ou nutritionnelle ultérieure, et qui sont susceptibles de respecter les conditions requises par les réglementations en vigueur pour la mise sur le marché d'une formulation pharmaceutique et/ou d'une formulation nutritionnelle.

Selon un aspect plus particulier, au moins un additif technologique comestible présent dans la composition (C_{A}), est un agent diluant, un agent d'écoulement, un agent liant ou un agent délitant.

Parmi les agents diluants que l'on peut associer dans la composition (C_{A}), on peut citer le lactose, le sucrose, le saccharose, le glucose, la maltodextrine, le mannitol, le sorbitol, le xylitol, l'isomalt, l'hydrogénophosphate de calcium, la cellulose microcristalline, les amidons et plus particulièrement les amidons de maïs, les amidons de blé, les amidons de pommes de terre, le phosphate dicalcique, le dibasic calcium phosphate anhydre, le carbonate de sodium, le carbonate de calcium et le carbonate de magnésium, les monoglycérides et/ou les diglycérides d'acides gras comportant de 8 à 24 atomes de carbone.

Parmi les agents d'écoulement que l'on peut associer dans la composition (C_{A}), on peut citer le stéarate de magnésium, le talc, le Stearyl Fumarate de sodium, les huiles végétales hydrogénées, la silice colloïdale anhydre, le benzoate de sodium, le dixoyde de silice.

Parmi les agents liants que l'on peut associer dans la composition (C_{A}), on peut citer les amidons sous forme d'empois, les amidons pré-gélatinisés, l'hydroxypropylméthyl cellulose, la methylcelllulose, les sirops de saccharose et la gomme d'acacia.

Parmi les agents délitant que l'on peut associer dans la composition (C_{A}), on peut citer les amidons, le glycolate d'amidon sodique, l'acide alginique, l'alginate de sodium, la croscarmellose sodique, la crospovidone, la polyvinylpyrrolidone.

Selon un aspect particulier, ce document a pour objet ladite composition comestible (C_{A}) telle que définie ci-dessus et consistant en, pour 100% de sa masse :
- De 5% à 50% massique, plus particulièrement de 5% à 40% massique, d'un sel d'un cation métallique multivalent (C_{M}) et d'un anion organique comestible,
- De 1% à 35% massique, plus particulièrement de 5% à 35% massique et tout particulièrement de 8% à 35% massique d'au moins un composé (V_{E}), choisi parmi la vitamine E ou l'acétate de vitamine E, d'acétate de vitamine E,
- De 0,5% à 80% massique, plus particulièrement de 1% à 70% massique d'une composition comestible (PP) de composés polyphénoliques choisis parmi les composés de la famille des flavanols, les composés de la famille des anthocyanes, les composés de la famille des acides phénoliques, et des composés de la famille des flavonols et/ou de leurs dérivés glucosylés,
- De 10% à 93,5% massique, plus particulièrement de 15% à 89% massique d'au moins un additif technologique comestible,
et dans laquelle le rapport molaire (C_{M})/(V_{E}) est supérieur ou égal à 0,50 et inférieur ou égal à 2,00, plus particulièrement supérieur ou égal à 0,50 et inférieur ou égal à 1,50.

La composition (C_{A}) peut se présenter sous toute forme physique, et plus particulièrement sous la forme d'une poudre.

Lorsque la composition (C_{A}) se présente sous la forme d'une poudre, elle est obtenue par introduction de ses différents constituants dans un mélangeur équipé d'au moins un système mécanique d'agitation, comme par exemple des pâles d'agitation plates ou de type hélice, et le mélangeur est éventuellement un mélangeur par retournement, et le mélangeur est éventuellement équipé d'un système de type émotteur. Cette opération de mélange est généralement réalisée à température ambiante.

Selon un aspect plus particulier, ce document a pour objet une composition (C_{A}) se présentant sous la forme d'une poudre et consistant pour 100% de sa masse :
- De 5% à 50% massique, plus particulièrement de 5% à 40% massique, de gluconate de zinc,
- De 1% à 35% massique, d'a-tocophérol,
- De 0,5% à 80% massique, de ladite composition (PP) 'une composition comestible (PP) comprenant de 60 % à 75 % massique d'au moins un composé de la famille des flavanols, de 10 % à 20 % massique d'au moins un composé de la famille des anthocyanes et de 5 % à 30 % massique d'au moins un composé de la famille des acides phénoliques ;
- De 10% à 93,5% massique, plus particulièrement de 15% à 89% massique d'au moins un additif technologique comestible,
et dans laquelle le rapport molaire (C_{M})/(V_{E}) est supérieur ou égal à 0,50 et inférieur ou égal à 2,00, plus particulièrement supérieur ou égal à 0,50 et inférieur ou égal à 1,50.

L'évaluation de la composition (C_{A}) telle que définie précédemment, par le procédé objet de la présente invention, fait apparaître que les rapports R₁, R₂ et R₃, tels que définis précédemment, sont tous les trois supérieurs à 40.

Selon un autre aspect, ce document a pour objet l'utilisation de la composition (C_{A}) telle que définie précédemment pour préparer une composition de complément alimentaire, ainsi que son utilisation comme complément alimentaire.

Par « composition de complément alimentaire », on entend au sens de la présente demande une composition dont le but est de fournir un complément de nutriments ou de substances possédant un effet nutritionnel ou physiologique, manquants ou en quantité insuffisante dans le régime alimentaire normal d'un individu.

Une composition de complément alimentaire constitue une source concentrée de nutriments ou d'autres substances ayant un effet nutritionnel ou physiologique seuls ou combinés, et permet d'éviter certaines carences ou de répondre à des besoins spécifiques dans le régime d'alimentation d'un individu, notamment lors d'un effort physique.

Les compositions de complément alimentaire répondent également à la définition telle que présente dans l'article 2 du Décret n° 2006-352 du 26 mars 2006 de la République Française relatif aux compléments alimentaires et/ou de la directive 2002/46/CE du parlement européen et du conseil du 10 juin 2002.

Par « composition alimentaire », on entend au sens de la présente demande une composition comprenant des aliments, à savoir toute substance ou tout produit transformé, partiellement transformé ou non transformé, destiné à être ingéré ou raisonnablement susceptible d'être ingéré par l'être humain. Les aliments répondent également à la définition présente dans le règlement européen 178/2002/CE.

Selon un autre aspect, ce document a pour objet une composition de complément alimentaire caractérisée en ce qu'elle comprend pour 100% de sa masse de 5 % à 70 % massique, plus particulièrement de 10 % à 70 % massique, et encore plus particulièrement de 25 % à 70 % massique de la composition (C_{A}) telle que définie précédemment.

Selon un aspect particulier, la composition de complément alimentaire se présente sous toute forme galénique connue de l'homme du métier, comme par exemple sous la forme d'un comprimé, d'une gélule, d'une capsule molle, d'un sirop, d'une poudre, comme par exemple une poudre à libération immédiate, d'une poudre à libération différée ou d'une poudre pour boissons reconstituées, d'un liquide, d'un stick, d'un gel.

Selon un autre aspect, ce document a pour objet une composition alimentaire caractérisée en ce qu'elle comprend pour 100% de sa masse de 5 % à 70 % massique, plus particulièrement de 10 % à 70 % massique, et encore plus particulièrement de 25 % à 70 % massique, de la composition (C_{A}) telle que définie précédemment.

Selon un aspect particulier, la composition alimentaire se présente sous toute forme de produit alimentaire connue de l'homme du métier, comme une boisson, et plus particulièrement une boisson aqueuse, une solution, un jus de fruit, une boisson aromatisée, une boisson énergétique, une boisson alcoolisée, une boisson à base de café, une boisson à base de chocolat, une boisson à base de thé, un produit laitier, et plus particulièrement du lait, du yaourt, un dessert lacté, du yaourt à boire, un fromage, une crème glacée, une barre chocolatée, un produit céréalier, et plus particulièrement une barre de céréale, un biscuit, des céréales pour le petit déjeuner, des farines, des produits de panification, un produit de nutrition spécialisée, plus particulièrement un produit de nutrition infantile, un produit de nutrition pour préparer à l'effort physique, un produit de nutrition clinique, un substitut de repas, des confiseries, plus particulièrement des gommes à mâcher, des bonbons, des caramels, des dragées, des berlingots, des guimauves, des lokoums, des nougats, des pâtes de fruits, des réglisses.

De façon générale, la composition de complément alimentaire peut comporter également des principes actifs habituellement mis en œuvre dans le domaine de la nutrition, comme des lipides bioactifs, les sels d'oligo-éléments hydrosolubles ou hydro-dispersibles, les vitamines hydrosolubles ou liposolubles, les pré-biotiques, les pro-biotiques, les protéines et/ou les concentrats de protéines laitières, les enzymes végétales ou animales, les acides aminés, les peptides, les sucres, les exhausteurs de goût, les agents aromatisants.

Comme lipides bioactifs éventuellement présents dans la composition de complément alimentaire, on peut citer les phytostérols, comme ceux extraits des huiles végétales, et plus particulièrement les extraits de l'huile d'argousier, de l'huile de maïs, de l'huile de soja ; les complexes de phytostérols, isolés des huiles végétales, comme par exemple la cholestatine, composée de campestérol, de stigmastérol et de brassicastérol ; les phytostanols ; les caroténoïdes, qui appartiennent à la famille des terpénoïdes, extraits des algues, des plantes vertes, des champignons, des bactéries ; les acides gras polyinsaturés du groupe oméga-3, comme par exemple l'acide alpha-linolénique, l'acide eicosapentaénoïque, l'acide docosahexanoïque ; les acides gras polyinsaturés du groupe oméga-6, comme par exemple l'acide linoléique, l'acide γ-linolénique, l'acide eicosadiénoïque, l'acide dihomo-γ-linolénique, l'acide arachidonique, l'acide docosadiénoïque, l'acide docosatétraénoïque, l'acide docosapentaénoïque.

Comme exemples de sels d'oligo-éléments hydrosolubles ou hydro-dispersibles éventuellement présents dans la composition de complément alimentaire, on peut citer le carbonate ferreux, le chlorure ferreux tétrahydraté, le chlorure ferrique hexahydraté, le citrate ferreux hexahydraté, le fumarate ferreux, le lactate ferreux tétrahydraté, le sulfate ferreux monohydraté, le sulfate ferreux heptahydraté, le chélate ferreux d'acides aminés hydratés, le chélate de fer de glycine ; l'iodate de calcium hexahydraté, l'iodate de calcium anhydre ; l'iodure de sodium, l'iodure de potassium ; l'acétate de cobalt tétrahydraté, le carbonate basique de cobalt monohydraté, le carbonate de cobalt hexahydraté, le sulfate de cobalt heptahydraté, le sulfate de cobalt monohydraté, le nitrate de cobalt hexahydraté ; l'acétate cuivrique monohydraté, le carbonate basique de cuivre monohydraté, le chlorure cuivrique dihydraté, le méthionate de cuivre, le sulfate cuivrique pentahydraté, le chélate cuivreux d'acides aminés hydratés, le chélate cuivreux de glycine hydraté, le chélate de cuivre de Phydroxy-analogue de méthionine ; le carbonate manganeux, le chlorure manganeux tétrahydraté, le phosphate acide de manganèse trihydraté, le sulfate manganeux tétrahydraté, le sulfate manganeux monohydraté, le chélate de manganèse d'acides amines hydraté, le chélate de manganèse de glycine hydraté, le chélate de manganèse de l'hydroxy-analogue de méthionine ; le molybdate d'ammonium, le molybdate de sodium, le sélénite de sodium, le sélénate de sodium ; la forme organique du sélénium produite par saccharomyces cerevisiae, la sélénomethionine (levure séléniée inactivée), et la séléno méthionine produite par Saccharomyces cerevisiae (levure séléniée inactivée).

Comme exemples de vitamines hydrosolubles ou liposolubles éventuellement présents dans la composition de complément alimentaire, on peut citer : la vitamine A, plus particulièrement sous sa forme de rétinol, d'acétate de rétinyle, de palmitate de rétinyle ou de bêta-carotène ; la vitamine D2, plus particulièrement sous sa forme dergocalciférol, ou de 25-hydrox calciférol, la vitamine D3, plus particulièrement sous sa forme de cholécalciférol, la vitamine K, plus particulièrement sous sa forme de phylloquinone (phytoménadione) ou de Ménaquinone, la vitamine B1, plus particulièrement sous sa forme de chlorhydrate de thiamine, de mononitrate de thiamine, de chlorure de thiamine monophosphate, ou de chlorure de thiamine pyrophosphate, la vitamine B2, plus particulièrement sous sa forme de riboflavine, de riboflavine 5'-phosphate de sodium, la vitamine B6, plus particulièrement sous sa forme de chlorhydrate de pyridoxine, de pyridoxine 5'-phosphate, ou de pyridoxal 5'-phosphate, la vitamine B12, plus particulièrement sous sa forme de cyanocobalamine, d'hydroxocobalamine, de 5'-déoxyadénosylcobalamine, ou de méthylcobalamine, la vitamine C, plus particulièrement sous sa forme d'acide L-ascorbique, de L-ascorbate de sodium, de L-ascorbate de calcium, de L-ascorbate de potassium, de sels de calcium de l'acide palmityl-6-L-ascorbique, de sodium ascorbylmonophosphate, l'acide pantothénique, plus particulièrement sous sa forme de D-pantothénate de calcium, de D-pantothénate de sodium, de Dexpanthénol, ou de Pantéthine, la vitamine PP, plus particulièrement sous sa forme d'acide nicotinique, de niacine, de nicotinamide, ou d'hexanicotinate d'inositol (hexaniacinate d'inositol), la vitamine B9, plus particulièrement sous sa forme d'acide folique, les folates, plus particulièrement sous leur forme d'acide ptéroylmonoglutamique, de L-méthyifolate de calcium, d'acide (6S)-5-méthyltétrahydrofolique sous forme de sel de glucosamine, la vitamine H2, B7 ou BW, plus particulièrement sous sa forme de biotine, la choline, plus particulièrement sous sa forme de chlorure de choline, de choline dihydrogène citrate, de bitartrate de choline, l'inositol, la carnitine, plus particulièrement sous sa forme de L-carnitine, L-carnitine-L-tartrate, la taurine.

Comme exemples de pré-biotiques éventuellement présents dans la composition de complément alimentaire, on peut citer l'inuline, les trans-galactooligosaccharides, les fructanes et les manno-oligosaccharides.

Comme exemples de pro-biotiques éventuellement présents dans la composition de complément alimentaire, on peut citer différentes souches de *Saccharomyces cerevisiae,* de *Bacillus cereus var toyoi,* de *Bacillus subtilis* seul ou en combinaison avec le *Bacillus licheniformis,* ou encore des souches *d'Enteroccocus faecium.*

Ces souches de microorganismes sont généralement associées à un support solide, par exemple le carbonate de calcium, le dextrose ou le sorbitol.

Comme exemples de protéines et/ou de concentrats de protéines éventuellement présents dans la composition de complément alimentaire, on peut citer les protéines laitières issues du cracking du lait, tels que le colostrum sous forme de poudre lyophilisée ou atomisée, le lactoserum sous forme de poudre, de fractions purifiées ou enrichies en IgG, en lactoferrine, en lactoperoxydase.

Comme exemples d'enzymes végétales ou animales éventuellement présents dans la composition de complément alimentaire, on peut citer la promutase, la superoxyde dismutase (SOD), la 3-phytase, la 6-phytase, les endo-l,4-betaglucanases, les endo-l,4- betaxylanases, ou encore d'autres enzymes améliorant ou favorisant la digestion.

Comme exemples de peptides éventuellement présents dans la composition de complément alimentaire, on peut citer les peptides de l'avocat, les peptides de lupin, les peptides de quinoa, les peptides de maca, les peptides de soja fermente ou non, les peptides de riz, les peptides présents dans l'extrait de graines d'Acacia macrostachya, les peptides présents dans les extraits de graines de passiflore.

Comme exemples d'acides aminés éventuellement présents dans la composition de complément alimentaire, on peut citer l'alanine, l'arginine, l'asparagine, l'acide aspartique, la cystéine, l'acide glutamique, la glutamine, la glycine, l'histidine, l'isoleucine, la leucine, la lysine, la méthionine, la phénylalanine, la proline, l'hydroxy-proline, la pyrrolysine, la sélméno-cystéine, la sérine, la thréonine, le tryptophane, la tyrosine, la valine, la sarcosine, l'ornithine.

Comme exemples de sucres éventuellement présents dans la composition de complément alimentaire, on peut citer les polysaccharides solubles dans l'eau, les sucres de poids moléculaire inferieur, tels que les oligosaccharides, mono- ou disaccharides, comme par exemple le glucose, le lactose, le dextrose.

Comme exemples d'exhausteurs de goût éventuellement présents dans la composition de complément alimentaire, on peut citer les glutamates, comme par exemple l'acide glutamique, le glutamate monosodique, le glutamate monopotassique, le diglutamate de calcium, le glutamate d'ammonium, le diglutamate de magnésium ; les guanylates, comme par exemple l'acide guanylique (guanosine monophosphate), le guanylate disodique, le Guanylate dipotassique, le Guanylate de calcium, les inosinates, comme par exemple l'acide inosinique, l'inosinate disodique, l'inosinate dipotassique, l'inosinate de calcium, ou encore des édulcorants intenses tels que les extraits de Stevia, les Rébiaudosides.

Selon un autre aspect, ce document a pour objet la composition comestible (C_{A}), telle que définie précédemment, pour la mise en œuvre d'une méthode de traitement thérapeutique du corps humain et plus particulièrement pour son utilisation pour prévenir la fatigue musculaire et/ou les dommages musculaires induits par un effort physique dans une méthode de traitement thérapeutique du corps humain.

L'exposé expérimental suivant illustre l'invention sans toutefois la limiter.

### A-1) - Choix des cellules et cinétique

### A-1-1) - Etape de mise en culture

Les cellules retenues pour la mise en œuvre du procédé objet de la présente invention, sont des cellules musculaires squelettiques primaires humaines, issues du muscle *gluteus maximus* (utilisé dans le cas d'effort musculaire chez l'homme), et en raison de leur capacité à se différencier en myotubes (ou fibres musculaires).

Pour évaluer leur capacité de différenciation, les cellules ont été mises en culture en plaques 48 puits à 10.000 cellules/puits, dans un milieu de culture spécifique, à 37°C dans une atmosphère humide contenant 5% de CO₂. Chaque condition est réalisée en quatre exemplaires (quadriplicate en langue anglaise)).

### A-1-2) - Etape de différenciation

Lorsque les cellules ont atteint 70% à 80% de confluence, leur milieu de culture a été remplacé par du milieu de différenciation, de façon à obtenir des myotubes. La différenciation des myoblastes en myotubes a été évaluée par la mesure de l'activité de la créatine kinase intracellulaire à l'issue de plusieurs durées, de façon à déterminer la durée optimale de l'étape de différenciation. L'activité de la créatine kinase intracellulaire est mesurée avec un kit de dosage par mesure spectrophotométrique à 340nm en cinétique. Les résultats obtenus sont exprimés en mU/mg de protéines.

Les résultats obtenus ont montré une activité optimale de la créatine kinase après une durée de 10 jours de différenciation, et que cette activité de la créatine kinase est plus importante pour un passage de cellules R3 que pour un passage de cellules R4.

L'étape de différenciation du procédé selon l'invention a donc été conduite pendant une durée de 10 jours pour un passage de cellules R3.

### A-1-3) - Etape de stress des cellules différenciées

Plusieurs concentrations de l'agent ionophore calcimycine (ou A23187) ont été testées sur les cellules différenciées obtenues à l'étape précédente.

Pour chacune de ces concentrations, la quantité de lactate, la quantité de l'Interleukine IL-6 et le niveau d'activité de la créatine kinase (CK) ont été évaluées.

La mesure de la quantité de lactate et de la quantité de l'Interleukine IL-6 a été réalisée par récupération des surnageants de chaque milieu de culture.

Le lactate a été quantifié avec un kit de dosage par mesure spectrophotométrique à 575nm, contre une gamme étalon de lactate. Les résultats ont été exprimés en pg/µg d'ADN.

L'IL-6 a été quantifiée avec un kit de dosage par mesure spectrophotométrique à 450nm, contre une gamme étalon d'IL-6. Les résultats ont été exprimés en pg/µg d'ADN.

Les tapis cellulaires ont été lysés dans du tampon PBS à l'aide d'une tige de sonication et 2 évaluations ont été réalisées sur ces derniers :
- Une évaluation de l'activité de la créatine kinase (CK) avec un kit de dosage par mesure spectrophotométrique à 340 nm en cinétique. Les résultats ont été exprimés en mU/µg d'ADN ; et
- Une évaluation de la quantité d'ADN par dosage fluorimétrique à l'aide d'un intercalant de l'ADN, le réactif Hoescht, contre une gamme étalon d'ADN.

Les résultats des quantités de lactate et d'IL-6 et les résultats de l'activité de la créatine kinase (CK) ont été exprimés relativement à la quantité d'ADN présente dans les tapis.

Une analyse statistique à l'aide du test de Student a été effectuée afin de comparer les quantités de lactate et d'IL-6 mesurées, et l'activité de la créatine kinase (CK) mesurée pour chaque concentration de calcimycine (ou A23187) testées et pour des cellules différenciées obtenues à l'étape précédente non associées à la calcimycine (ou A23187).

Les résultats obtenus ont permis de montrer que la concentration en calcimycine (ou A23187) optimale est 1 µ mole/litre (ou 1µM).

### A-2) - Définition du procédé optimal.

Le procédé comprend donc les étapes suivantes :
- Etape a) de mise en culture de cellules musculaires squelettiques primaires humaines, issues du muscle *gluteus maximus* selon les conditions opératoires décrites dans le paragraphe A-1-1) ci-dessus,
- Etape b) de différenciation cellulaire du milieu obtenu à l'étape a) selon les conditions opératoires décrites dans le paragraphe A-1-2) ci-dessus, et notamment pendant une durée de 10 jours pour un passage de cellules R3,
- Etape c) de mise en contact de mise en contact du milieu de culture obtenu à l'étape b) avec la substance ou la composition à tester,
- Etape d) de mise en contact du milieu obtenu à l'étape c) avec la calcimycine (ou A23187) à une concentration de 1µmole/litre (ou 1µM),
- Etape e) de mesure de la quantité de lactate et de la quantité de l'Interleukine IL-6 sur le surnageant du milieu de culture et de mesure de l'activité de la créatine kinase (CK) selon les méthodes décrites dans le paragraphe A-1-3) ci-dessus.

Etape f) de comparaison des niveaux d'expression de la créatine kinase (CK) de l'IL-6 et du lactate. De façon, plus précise, on calcule dans le cadre de cette étape f) :
Le rapport R₁ = [(N¹₀ - N¹i) x 100] / [(N¹₀ - N¹)] pour l'interleukine IL-6 extracellulaire avec :
   N¹ correspondant à la quantité d'IL-6 mesurée dans le milieu de culture obtenue à l'issue du procédé tel que décrit ci-dessus, sans mettre en œuvre ni l'étape c) et ni l'étape d) du procédé (cellules différenciées non traitées et non stressées),
   N¹₀ correspondant à la quantité d'IL-6 mesurée dans le milieu de culture obtenue à l'issue du procédé tel que décrit ci-dessus, sans mettre en œuvre l'étape c) du procédé (cellules différenciées non traitées mais stressées),
   N¹i correspondant à la quantité d'IL-6 mesurée dans le milieu de culture obtenue à l'issue du procédé tel que décrit ci-dessus, lorsque une substance ou une composition (i) est mise en œuvre dans l'étape c) du procédé (cellules différenciées traitées et stressées) ;
Le rapport R₂= [(N²₀ - N²i) x 1001 / [(N²₀ - N²)] pour la créatine kinase intracellulaire avec :
   N² correspondant à l'activité créatine kinase mesurée dans le milieu de culture obtenue à l'issue du procédé tel que décrit ci-dessus, sans mettre en œuvre ni l'étape c) et ni l'étape d) du procédé (cellules différenciées non traitées et non stressées),
   N²₀ correspondant à l'activité créatine kinase mesurée dans le milieu de culture obtenue à l'issue du procédé tel que décrit ci-dessus, sans mettre en œuvre l'étape c) du procédé (cellules différenciées non traitées mais stressées),
   N²i correspondant à l'activité créatine kinase mesurée dans le milieu de culture obtenue à l'issue du procédé tel que décrit ci-dessus, lorsque une substance ou une composition (i) est mise en œuvre dans l'étape c) du procédé (cellules différenciées traitées et stressées) ;
Le rapport R₃ = [(N³₀ - N³i) x 100] / [(N³₀ - N³)] pour le lactate extracellulaire avec :
   N3 correspondant à la quantité de lactate mesurée dans le milieu de culture obtenue à l'issue du procédé tel que décrit ci-dessus, sans mettre en œuvre ni l'étape c) et ni l'étape d) du procédé (cellules différenciées non traitées et non stressées),
   N³₀ correspondant à la quantité de lactate mesurée dans le milieu de culture obtenue à l'issue du procédé tel que décrit ci-dessus, sans mettre en œuvre l'étape c) du procédé (cellules différenciées non traitées mais stressées),
   N³i correspondant à la quantité de lactate mesurée dans le milieu de culture obtenue à l'issue du procédé tel que décrit ci-dessus, lorsque une substance ou une composition (i) est mise en œuvre dans l'étape c) du procédé (cellules différenciées traitées et stressées).

### B1- Evaluation de substances et de compositions selon le procédé objet de la présente invention.

La composition (C₁) a été préparée par mélange de ses différents ingrédients constitutifs par introduction successive de ses ingrédients constitutifs dans un mélangeur équipé d'un système mécanique d'agitation équipé de pâles d'agitation plates et de pâles de type hélice, à une température de 25°C.

La composition C₁ consiste pour 100% de sa masse en :
38,2% de gluconate de zinc,
31,0 % massique d'acétate de D-α tocophérol,
23,18% massique d'amidon de maïs,
6,02% massique de dioxyde de silice,
1,6% massique d'un résidu sec de vin rouge, commercialisé sous le nom Provinol™, et comprenant une teneur en composés polyphénoliques de 90% massique ;
Les résultats obtenus par la mise en œuvre du procédé d'évaluation objet de la présente demande de brevet, sont consignés dans le Tableau 1 ci-dessous :

**Tableau 1**

| Produit testé (% massique) | IL-6 extracellulaire (pg/µg d'ADN) | Activité créatine kinase intracellulaire (pg/µg d'ADN) | Lactate extracellulaire (pg/µg d'ADN) |
|---|---|---|---|
| Cellules différenciées non traitées et non stressées | N¹ = 3,95±1,56 | N² = 38,07±5,36 | N³ = 14,62±1,87 |
| Cellules différenciées non traitées et stressées | N¹₀ = 12,29±1,43 | N²₀ = 16,86±8,04 | N³₀ = 24,92±4,77 |
| Cellules différenciées traitées avec le Provinol™^{(*)} (0,00005%) et stressées | N¹ᵢ = 11,47 ±1,42 R₁ = 10 | N²ᵢ = 12,66±2,16 R₂ = -20 | N³ᵢ = 25,13±1,74 R₃ = -2 |
| Cellules différenciées traitées avec le GlnZn^{(**)} (0,00015% en Zn) et stressées | N¹ᵢ = 12,44 ± 1,80 R₁ = -2 | N²ᵢ = 10,73 ± 2,28 R₂ = -29 | N³ᵢ = 28,33±6,43 R₃ = -33 |
| Cellules différenciées traitées avec la Vitamine E^{(***)} (0,001%) et stressées | N¹ᵢ = 16,55 ± 5,99 R₁ = -51 | N²ᵢ = 26,88 ± 2,82 R₂ = 47 | N³ᵢ = 27,34±3,43 R₃ = -24 |
| Cellules différenciées traitées avec (C₁) (0,001%) et stressées | N¹ᵢ = 7,00±0,72 R₁ = 63 | N²ᵢ = 33,24 ± 9,35 R₂ = 77 | N³ᵢ = 16,12±4,59 R₃ = 85 |

| | | | |
|---|---|---|---|
| (*) : le Provinol™ est un résidu sec de vin rouge, comprenant une teneur en composés polyphénoliques de 90 % massique pour 100% de sa masse soit une proportion massique en composés polyphénoliques de 1,44%. (**) : Le gluconate de zinc comprend une teneur en cation divalent du zinc de 12,2 % (***) : la Vitamine E est de l'acétate de D-α tocophérol | | | |

### Analyse des résultats

Les rapports R₁, R₂ et R₃ sont inférieurs à 40, pour les cellules traitées au Provinol™ (résidu sec de vin rouge à base de composés polyphénoliques). Il en est de même pour les cellules traitées au gluconate de zinc. On en déduit que ni le Porvinol™, ni le gluconate de zinc et par conséquent, le cation divalent du zinc, ne peuvent être sélectionné seuls pour prévenir la fatigue musculaire et les dommages musculaires induits par un effort physique chez l'être humain.

Les rapports R₁ et R₃ sont inférieurs à 40, et le rapport R₂ supérieur à 40 (R₂ = 47) pour les cellules traitées avec l'acétate de D-α tocophérol. Ceproduit ne peut donc pas non plus être sélectionné pour prévenir la fatigue musculaire et les dommages musculaires induits par un effort physique chez l'être humain.

Au contraire les rapports R₁, R₂ et R₃ sont tous supérieurs à 40, pour des cellules traitées avec la Composition (C₁).

Cette composition (C₁) peut être sélectionnée pour prévenir la fatigue musculaire et les dommages musculaires induits par un effort physique chez l'être humain.

## Revendications

1. Procédé pour évaluer la capacité d'une substance chimique (S) ou d'une composition chimique (C) à prévenir la fatigue musculaire et les dommages musculaires induits par un effort physique chez l'être humain, ledit procédé étant **caractérisé en ce qu'**il comprend :
- Une étape a) de mise en culture de cellules musculaires squelettiques primaires humaines ;
- Une étape b) de différenciation des cellules obtenues à l'étape a) pour obtenir des myotubes ;
- Une étape c) de mise en contact d'une substance chimique (S) ou d'une composition chimique (C) avec le milieu cellulaire obtenu à l'étape b) ;
- Une étape d) de mise en contact d'au moins un agent ionophore de calcium (Al) avec le milieu cellulaire obtenu à l'étape c) ;
- Une étape e) de mesure du niveau d'expression d'une combinaison de marqueurs biologiques comprenant ou consistant en :
- Au moins un marqueur biologique (M₁) de l'homéostasie glycémique choisi parmi les éléments du groupe constitué par les myokines ou cytokines produites par les muscles,
- Au moins un marqueur biologique (M₂) des lésions musculaires choisi parmi la créatine kinase et la lactate déshydrogénase,
- Au moins un marqueur biologique (M₃) du métabolisme de l'ATP choisi parmi les éléments du groupe constitué par le lactate, l'ammoniaque, les oxypurines,
dans le milieu de culture obtenu à l'étape d) ; et
- Une étape f) de comparaison des niveaux d'expression de chacun desdits trois marqueurs biologiques (M₁), (M₂) et (M₃) mesurés à l'étape e) avec le niveau d'expression de référence pour chacun de ces trois marqueurs biologiques.

2. Procédé tel que défini à la revendication 1, **caractérisé en ce que** dans l'étape d), l'agent ionophore de calcium (Al) est sélectionné parmi les éléments du groupe constitué par la beauvéricine, l'ionomycine, la calcimycine (ou A23187).

3. Procédé tel que défini à la revendication 2, **caractérisé en ce que** l'agent ionophore de calcium (Al) est la calcimycine.

4. Procédé tel que défini à l'une ou quelconque des revendications 1 à 3, **caractérisé en ce que** le marqueur biologique (M₁) est l'Interleukine-6, le marqueur biologique (M₂) est la créatine kinase et le marqueur biologique (M₃) est le lactate.

## Patentansprüche

1. Verfahren zum Beurteilen der Fähigkeit einer chemischen Substanz (S) oder einer chemischen Zusammensetzung (C), die Muskelermüdung und die durch eine körperliche Anstrengung verursachten Muskelschäden beim Menschen zu verhindern, wobei das Verfahren **dadurch gekennzeichnet ist, dass** es umfasst:
- einen Schritt a) des Kultivierens von primären menschlichen Skelettmuskelzellen;
- einen Schritt b) des Differenzierens der in Schritt a) erhaltenen Zellen, um Myotuben zu erhalten;
- einen Schritt c) des In-Kontakt-Bringens einer chemischen Substanz (S) oder einer chemischen Zusammensetzung (C) mit dem in Schritt b) erhaltenen Zellmilieu;
- einen Schritt d) des In-Kontakt-Bringens mindestens eines Calcium-Ionophor-Mittels (AI) mit dem in Schritt c) erhaltenen Zellmilieu;
- einen Schritt e) des Messens des Expressionsniveaus einer Kombination von biologischen Markern, umfassend oder bestehend aus:
- mindestens einem biologischen Marker (M₁) der glykämischen Homöostase, ausgewählt aus den Elementen der Gruppe, bestehend aus den durch die Muskeln produzierten Myokinen oder Zytokinen,
- mindestens einem biologischen Marker (M₂) der muskulären Läsionen, ausgewählt aus Kreatinkinase und Lactatdehydrogenase,
-- mindestens einem biologischen Marker (M₃) des ATP-Metabolismus, ausgewählt aus den Elementen der Gruppe, bestehend aus Lactat, Ammoniak, Oxypurinen,
in dem in Schritt d) erhaltenen Zellmilieu; und
- einen Schritt f) des Vergleichs der Expressionsniveaus von jedem der drei in Schritt e) gemessenen biologischen Markern (M₁), (M₂) und (M₃) mit dem Referenzexpressionsniveau für jeden dieser drei biologischen Marker.

2. Verfahren nach Anspruch 1, **dadurch gekennzeichnet, dass** im Schritt d) das Calcium-Ionophor-Mittel (AI) ausgewählt ist aus den Elementen der Gruppe, bestehend aus Beauvericin, lonomycin, Calcimycin (oder A23187).

3. Verfahren nach Anspruch 2, **dadurch gekennzeichnet, dass** das Calcium-Ionophor-Mittel (AI) Calcimycin ist.

4. Verfahren nach einem der Ansprüche 1 bis 3, **dadurch gekennzeichnet, dass** der biologische Marker (M₁) das Interleukin-6 ist, der biologische Marker (M₂) die Kreatinkinase ist und der biologische Marker (M₃) das Lactat ist.

## Claims

1. Method for evaluating the ability of a chemical substance (S) or of a chemical composition (C) to prevent muscle fatigue and muscle damage induced by physical exertion in humans, said method being **characterised in that** it comprises:
- a step a) of culturing human primary skeletal muscle cells;
- a step b) of differentiating the cells obtained in step a) in order to obtain myotubes;
- a step c) of bringing a chemical substance (S) or a chemical composition (C) into contact with the cell medium obtained in step b) ;
- a step d) of bringing at least one calcium ionophore agent (Al) into contact with the cell medium obtained in step c);
- a step e) of measuring the level of expression of a combination of biological markers comprising or consisting of:
- at least one biological marker (M₁) of glycaemic homeostasis, chosen from the elements of the group consisting of the myokines or cytokines produced by muscles,
- at least one biological marker (M₂) of muscle lesions, chosen from creatine kinase and lactate dehydrogenase,
- at least one biological marker (M₃) of ATP metabolism, chosen from the elements of the group consisting of lactate, aqueous ammonia and oxypurines, in the culture medium obtained in step d); and
- a step f) of comparing the levels of expression of each of said three biological markers (M₁), (M₂) and (M₃) measured in step e) with the reference expression level for each of these three biological markers.

2. Method as defined in claim 1, **characterised in that**, in step d), the calcium ionophore agent (Al) is selected from the elements of the group consisting of beauvericin, ionomycin and calcimycin (or A23187).

3. Method as defined in claim 2, **characterised in that** the calcium ionophore agent (Al) is calcimycin.

4. Method as defined in any of claims 1 to 3, **characterised in that** the biological marker (M₁) is interleukin-6, the biological marker (M₂) is creatine kinase and the biological marker (M₃) is lactate.
